# EUROPEAN PATENT APPLICATION

(11) **EP 4 538 652 A1**
(43) Date of publication of application: **16.04.2025**
(21) Application number: 24205401.3
(22) Date of filing: 08.10.2024
(51) Int. Cl.: G01F 1/58, G01N 33/18, C02F 1/00, G01N 27/416

(54) **MEASUREMENT APPARATUS**

(30) Priority: 12.10.2023 GB 202315666; 09.02.2024 GB 202401774
(71) Applicant: Sensus Spectrum LLC., Morrisville, NC 27560 (US)
(72) Inventor: BROOM, Michael, Cambridge, CB5 8NB (GB); DI PIETRO, Riccardo, Cambridge, CB4 3QQ (GB); MEANWELL, Hilary, Saffron Walden, CB10 2AN (GB)
(74) Representative: Piotrowicz, Pawel Jan Andrzej

(57) **Abstract**

Apparatus (1) for monitoring a fluid stream is disclosed. The apparatus, comprising a measurement section (3), comprising a flow tube (4) having a flow passage (6), a quasi-reference electrode (12) in fluid communication with the flow passage, a working electrode (13) in fluid communication with the flow passage, a circuit (14) configured to measure the potential difference between the quasi-reference electrode and the working electrode; and an interface (15) for outputting the potential difference. The quasi-reference electrode comprises a coated metal electrode (21; Fig. 2) consisting of a metal electrode (22; Fig. 2) having a surface which at least part of which is coated with a layer (23; Fig. 2) of an insoluble salt of the metal.

## Description

### Field

The present invention relates to measurement apparatus.

### Background

The oxidation-reduction potential (ORP) of a solution measures the ability of the solution to oxidise or reduce another chemical substance. Each chemical species in the solution has its own oxidation-reduction potential (also referred to as "redox potential") and the overall ORP of the solution is determined by a stable point where all the current contributions due to the ORP potentials of the different species are balanced.

In water networks (both drinking water and waste water) and treatment plants, ORP is regularly used to monitor the different stages of treatment and distribution. It can be used, among other things, for dosing control (for example, in chlorine dosing) and for contamination detection and process control.

ORP can be monitored to ensure the correct dosing of disinfectant. When combined with pH measurements it can be used for the absolute estimation of the chlorine concentration in pool water. ORP is not a selective measurement and is affected by any molecule present in the water being tested which is electrochemically active (capable of donating or collecting electrons and form stable ions).

ORP is determined by measuring the potential difference between a reference electrode (such as the standard hydrogen electrode or the Ag/AgCI/KCI reference electrode) and a working electrode. The reference electrode provides a stable reference potential (determined by the half-cell reaction taking place on its surface) against which the potential of the working electrode is measured.

The working electrode is made of an inert material such as gold, platinum or carbon so that no unwanted reaction takes place at its surface. The measured voltage is then determined by the redox potential of the sample under test. The measurement is performed using a high input impedance voltmeter to minimise leakage current which can cause unwanted electrochemical reactions, thus affecting the measured voltage and changing the composition of both the electrodes and the sample under test. Commercially-available ORP sensors are based on the measurement principle hereinbefore described which allows to relate the absolute value of the measured potential to the properties of the liquid being tested. This type of measurement, however, has practical limitations.

First, maintaining a stable reference potential requires frequent renewal of the reference electrode. Operations include replacing the electrolyte (KCI solution in a Ag/AgCI/KCI reference, or HCl in a standard hydrogen electrode) which tends to be carried out by a trained technician. Secondly, it tends to involve frequent calibration against a reference standard. Since the accuracy of the measurement can be easily affected by the conditions at the surface of the two electrodes, operation has to be checked and calibrated against reference solutions to ensure proper operation. Thirdly, due to the complexity introduced by the reference electrodes (for example, the porous glass frit in a Ag/AgCl/KCl, or the pressure requirements in a SHE), these sensors are usually limited in their operational ratings and installation requirements. Most of these sensors are installed in a "side-stream" configuration where liquid from the main stream is sent to a dedicated line, reduced in pressure before being measured by the sensor. The liquid is then discarded and treated as waste.

These limitations have hitherto discouraged or prevented widespread adoption of ORP measurement for monitoring distribution networks. Where stricter pressure regulations are in place, it is not always possible to find a suitable site for "side-stream" installation and regular maintenance schedule would make the operation of the sensor highly expensive.

Reference is made to US 2022/0153616 A1 which describes a system for monitoring disinfectant levels in non-human animal drinking water using ORP.

### Summary

According to a first aspect of the present invention there is provided apparatus, comprising a measurement section, comprising a flow tube having a flow passage. The apparatus further comprises a quasi-reference electrode in fluid communication with the flow passage, a working electrode in fluid communication with the flow passage, a circuit configured to measure the potential difference between the quasi-reference electrode and the working electrode and an interface for outputting the potential difference. The quasi-reference electrode comprises a coated metal electrode consisting of a metal electrode having a surface which at least part of which is coated with a layer of an insoluble salt of the metal.

A salt is considered herein to be "insoluble" in a solvent if less than 0.1 g of the salt dissolves in 100 mL of the solvent, e.g., water, at room temperature (298 K) and room pressure (101 kPa).

The metal may be silver, and the insoluble salt may be silver chloride.

The quasi-reference electrode may further comprise a porous element, wherein the porous element is interposed between the coated metal electrode and the flow tube so as to provide a fluid path between the flow tube and the coated metal electrode. The porous element may be a ceramic porous element. The porous element may be graphite porous element.

The working electrode may comprise an inert, electrically-conductive material electrode at least partially disposed within a passage at an end which is proximate to the flow passage. The inert, electrically-conductive material may comprise a noble metal, such as platinum or gold, carbon, for example, in the form of graphite. The inert, electrically-conductive material electrode may be porous.

The apparatus may further comprise an electrically-conductive connector in direct contact with the electrically-conductive material electrode, wherein the inert, electrically-conductive material electrode is interposed between the electrically-conductive connector and the flow passage. The electrically-conductive connector may comprise, or be formed of, an electrically-conductive polymer. The electrically-conductive connector may be configured to provide a fluid-tight seal in the passage.

The flow tube may include an aperture, and the quasi-reference electrode and the working electrode may be disposed in the aperture. The flow tube may include more than one aperture.

The circuit may be provided by a processing unit of a water meter.

The apparatus may be configured to be electromagnetic flow type water meter.

The apparatus may further comprise a further electrode in fluid communication with the flow passage, wherein the working electrode and further electrode are spaced apart for electromagnetic flow sensing. The working electrode may comprise an inert, electrically-conductive material electrode and the further electrode comprising an inert, electrically-conductive material electrode, and the working electrode and the and further electrodes are disposed on opposite sides of the flow tube. The quasi-reference electrode may be a first quasi-reference electrode and the further electrode is a second quasi-reference electrode comprising a coated metal electrode consisting of a metal electrode having a surface which at least part of which is coated with a layer of an insoluble salt of the metal, wherein the first and second quasi-reference electrodes are disposed on opposite sides of the flow tube.

The working electrode and the quasi-reference electrode may be disposed on opposite sides of the flow tube.

The fluid may be a liquid. The liquid may be water, such as drinking water, for example, for human consumption. The fluid may be a gas.

The apparatus may be configured to measure the potential difference periodically (for instance, every 24 hours), according to a schedule (which may be aperiodic), on demand (which may be triggered locally or remotely), and/or in response to a given flow condition. The given flow condition may be exceeding a given flow rate and/or exceeding a given accumulated volume of flow of fluid.

According to a second aspect of the present invention there is provided a water meter comprising the apparatus of the first aspect and apparatus for measuring fluid flow through the flow tube.

The apparatus for measuring fluid flow through the flow tube may be an electromagnetic flow type water meter.

According to a third aspect of the present invention there is provided a system comprising a water meter, and the apparatus of the first aspect, wherein the fluid meter and the apparatus are discrete units arranged such that fluid is communicated through the meter and then through the apparatus or *vice versa.*

The water meter may be an electromagnetic flow type water meter.

According to a fourth aspect of the present invention there is provided a computer system, comprising at least one processor, memory, and a network interface. The at least one processor is configured, in response to receiving a set of measurements from a set of remote water meters at known locations relating to oxidation reduction potential, discoloration and/or turbidity to detect a change in water being delivered to a given water meter and/or to a given sub-set of meters, and/or to determine a cause of a change in water being delivered to a given water meter and/or to a given sub-set of meters, and/or to determine a statistical estimate of a point of origin in a water network resulting in a change in water being delivered to a given water meter and/or to a given sub-set of meters, and/or to determine a severity of change in water being delivered to a given water meter and/or to a given sub-set of meters.

The at least one processor may be configured to combine the flow data with the ORP and/or discolouration and/or turbidity data. The at least one processor may be configured to combine the water pressure data with ORP and/or discolouration and/or turbidity data.

According to a fifth aspect of the present invention there is provided a drinking water delivery system, comprising a set of remote water meters provided with or including a respective apparatus of the first aspect and deployed through a water supply network, and the computer system of the fourth aspect, wherein the computer system is arranged to communicate with the set of remote water meters so as to receive the set of measurements.

The remote water meters and the computer system may be configured to communicate via wireless communication links. The water meter may be configured to control the apparatus and instruct the apparatus to perform a measurement.

The apparatus may be configured to perform a measurement according to a schedule and to report the result of the measurement. The computer system may be configured to instruct at least one of the water meters to capture and report an additional measurement based on the values already stored in the system based on the values stored in the centralised storage system.

According to a sixth aspect of the present invention there is provided an apparatus for monitoring a fluid stream comprising a measurement section with an inlet and an outlet, to be placed in-line with the main fluid stream, so that all the fluid from the main fluid stream enters the inlet and is then returned from the outlet to the main fluid stream, a quasi-reference electrode (QRE) in contact with the fluid stream being measured, a working electrode in contact with the fluid stream being measured and placed within the same measurement section as the QRE, an electronic circuit measuring the potential difference between the two electrodes, a means to communicate the measured potential difference to a main processing unit.

The fluid may be drinking water. The measurement section may be shared with the measurement section of a water meter. The measurement section may be placed next to the measurement section of a water meter.

The quasi-reference electrode may be composed of a silver (Ag) wire anodised with silver chloride (AgCl) in contact with the water stream. The QRE may be covered by a ceramic element (electrically insulating) or graphite element porous to the fluid stream. The working electrode may be made out of an inert conductor such as platinum or gold or graphite or carbon. The electronic circuit may be part of the water meter main processing board. The water meter may be of the electromagnetic flow type. The working electrode may also act as one of the electrodes used to detect flow according to the electromagnetic flow sensing method.

According to a seventh aspect of the present invention there is provided a drinking water delivery system comprising the apparatus of the first and/or seventh aspect, a collection of fiscal water meters installed in a water supply network wherein: at least some meters are capable of electronically communicating to a centralised storage and analysis unit; at least some meters have at least one additional sensor capable of measuring at least one property of the water; wherein the sensor is mounted within the water meter body and/or in line with the water meter in the main water stream, measuring at least one of the following ORP (oxidation reduction potential), discolouration, turbidity; and a centralised storage and analysis unit that uses stored data to detect changes in the water being delivered to a water meter and/or a group of meters and/or inferring the cause of the change and/or providing a statistical estimate of the origin point in the network and/or the severity of changes in the water being delivered.

The centralised storage and analysis unit may combine the flow data with the ORP and/or discolouration and/or turbidity data. The centralised storage and analysis unit may combine the water pressure data with the ORP and/or discolouration and/or turbidity data. The communication between the water meter and the centralised storage and analysis unit may use wireless communication protocols. The meter may have the capability of controlling the water quality sensor and request the measurement to be performed. The water quality sensor may measure independently at regular intervals and reports the information to the water meter. The centralised storage system can request at least one of the water meters to capture and can report an additional measurement based on the values already stored in the system based on the values stored in the centralised storage system.

### Brief Description of the Drawings

Certain embodiments of the present invention will now be described, by way of example, with reference to the accompanying drawings in which:
Figure 1 is a schematic side view of a sensor;
Figure 2 is a schematic side view of a quasi-reference electrode used in the sensor shown in Figure 1;
Figure 3 is an exemplary plot of a measurement of ORP using an Ag/AgCI electrode as quasi-reference electrode;
Figures 4A, 4B and 4C are plots of QRE potential against a laboratory-grade Ag/AgCI/KCI(3M) reference electrode;
Figure 5 is a schematic side view of a unitary sensor and flow meter;
Figure 6 is a schematic side view of another quasi-reference electrode;
Figure 7 is a schematic diagram of a water delivery network; and
Figure 8 is a schematic diagram of a part of a water delivery network.

### Detailed description of Certain Embodiments

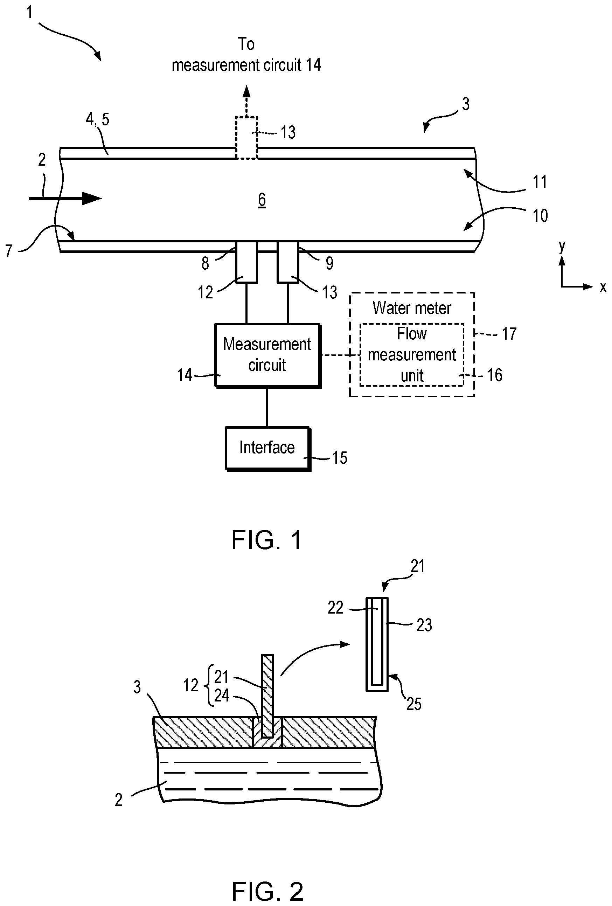

Referring to Figure 1, apparatus 1 (or a "sensor" or "monitor") for measuring oxidation-reduction potential (ORP) of a fluid 2 (Figure 2) is shown. In this case, the fluid 2 (Figure 2) is water, in particular, drinking water and so the fluid is hereinafter referred to as water.

The apparatus 1 comprises a measurement section 3 in the form of a section of pipe 4 (herein also referred to as a "conduit" or "flow tube") between an inlet (not shown) and an outlet (not shown). The apparatus 1 is used for an in-line measurement in which the measurement section 3 samples all of the water flowing through the main flow and thus the conduit 4 has a suitably large inner diameter. Expressed differently, the apparatus 1 is not used for a side-stream or measurement in which either fluid is taken from the main flow to fill a measurement chamber, a measurement is taken and the fluid is then discarded, or for a bypass measurement in which fluid enters into a smaller parallel channel in which a measurement is taken and then the fluid is returned to the main stream. The pipe 4 has a side wall 5, which defines a flow passage 6 (or "lumen"), having an inner surface 7. The pipe 4 has first and second apertures 8, 9 (or "ports") through the side wall 5 on a first side 10 of the pipe 3, spaced apart along the pipe 4.

A quasi-reference electrode 12 and a working electrode 13 are seated in the first and second apertures 8, 9, respectively so as to be in fluid communication with the flow passage 6. Thus, when water 2 (Figure 2) flows through the flow passage 6, the electrodes 12, 13 are wetted. The second aperture 9 and the working electrode 13 may, however, be disposed anywhere within the measurement section 3. For example, the second aperture 9 and the working electrode 13 may be disposed on the second side 11 of the pipe 3, opposite to the first side of the pipe 4.

Alternatively, the quasi-reference electrode 12 and the working electrode 13 can be disposed on the same aperture 8. For example, a single insert (not shown) can take the form of a central metal pin (not shown) which provides the quasi reference electrode, a second, concentric annular ring (not shown) which provides the working electrode, and a concentric dielectric ring interposed between the central pin and annular pin so as to provide an electrically-insulating spacer. Other arrangements of electrodes 12, 13 can be used, for example, in which the electrodes are seated within an electrically-insulating support (not shown) and the assembly is disposed in a single aperture in the measurement section. The electrodes 12, 13 can be placed behind a porous element 24, in other words, the porous element is interposed between the the flow passage 6 and the electrodes 12, 13. Alternatively, only the quasi-reference electrode 12 is placed behind the porous element 24. The electrodes 12, 13 may be screen printed on an electrically-insulating substrate (not shown).

The apparatus 1 also includes circuit 14 for measuring the potential difference V between the electrodes 12, 13 and an interface 15, *e.g.,* a wired bus or a wireless communications network interface, for outputting the measured potential difference V.

The value may be passed from the circuit 14 directly or via the interface 15 to a flow measurement unit 16 of a water meter 17. The apparatus 1 and the water meter 17 may be separate units (for example, having different measurement sections), may share the same measurement section, or may share additional components (such as at least one electrode 12, 13). For example, quasi-reference electrode 12 and the working electrode 13 can be shared in the same measurement section of a water meter 17. Alternatively, the quasi-reference electrode 12 or the working electrode 13 can be shared in the same measurement section of a water meter 17. In some embodiments, the quasi-reference electrode 12 or the working electrode 13 are used for both flow and ORP sensing. As will be explained in more detail hereinafter, the apparatus 1 may be incorporated into a fluid meter capable of measuring the flow rate of the fluid and sharing the same measurement section 3.

The apparatus 1 uses a quasi-reference electrode 12 instead of a reference electrode and is wetted by the liquid being tested via a porous membrane (which may be electrically conductive or non-conductive) rather than using a dedicated electrolyte. A quasi-reference electrode is a type of reference electrode which is used in specific instances where aqueous electrolytes cannot be used. It is characterised by being an electrode with a stable potential during a period of the measurement (e.g., between a day and a week, or more than a week), but for which the potential value is unknown.

The measurement section 3 can be placed in-line with the main fluid stream so that all the fluid from the main fluid stream enters the inlet and is then returned from the outlet to the main fluid stream.

Referring to Figure 2, the quasi-reference electrode 12 comprises a coated metal electrode 21 consisting of a metal electrode 22 coated with a layer 23 of an insoluble salt of the metal, at least partially inserted or embedded in a porous element 24. The porous element 24 allows the fluid 2 to come into direct contact with the coated metal electrode 21 but limits or prevents the electrode 21 being contaminated by the fluid, thereby extending the electrode lifetime, and slowing the diffusion of the fluid 2 from the measurement section to the surface 25 of the electrode 21.

In this case, coated metal electrode 21 takes the form of a silver wire (or "rod") anodised with silver chloride (AgCI). The half-cell reaction can be expressed as:

AgCl(s) + 1e⁻ ⇆ Ag(s) + Cl⁻(aq)

Due to the electrolyte not being the standard 3M KCI solution, the concentrations of the different species involved in the half-cell reaction are unknown. Thus, the potential of the electrode against a standard hydrogen electrode (SHE) is not well defined. Other salts of silver can be used, such as silver nitrate (AgNOs) or silver sulphate (Ag₂SO₄). Other metals can be used, such as copper, and the salt of copper may be copper sulphate (CuSO₄)

The porous element 24 consists of a ceramic. Other materials, however, can be used, such as graphite or plastic. The porous element 21 can be electrically conductive or non-conductive. The porous element allows the fluid 2 to diffuse to the electrode 21, but prevents excessive flow from reaching the electrode 16 which might otherwise strip the anodic coating 23.

The porous element 24 has the effect of slowing down diffusion of chemical species from the solution, i.e., fluid 2, to the electrode surface 25, enhancing the stability of the quasi-reference electrode 12 during the measurement time.

The working electrode 13 is made of an inert conductor, such as platinum (Pt), gold (Au), amorphous carbon (a-C) or porous or non-porous graphite.

The two electrodes 12, 13 are connected to an electronic sampling circuit 14 which has a high input impedance (>1 GΩ) to limit the leakage current through the two electrodes 12, 13 and reduce any unwanted electrochemical reaction.

A sensor 1 using a quasi-reference electrode 12 instead of a well-defined reference electrode cannot be used for a direct measurement of the ORP of the liquid 2 because the potential of the reference electrode is unknown. A property of the quasi-reference electrode 12 is, however, that its potential is stable over a prolonged periods of time unless the bulk composition of the water being tested is changed.

The sensor 1 can measure sudden changes (<1h) in ORP with similar accuracy to a standard ORP sensor, with an in-line installation compatible such as the one of a domestic water meter.

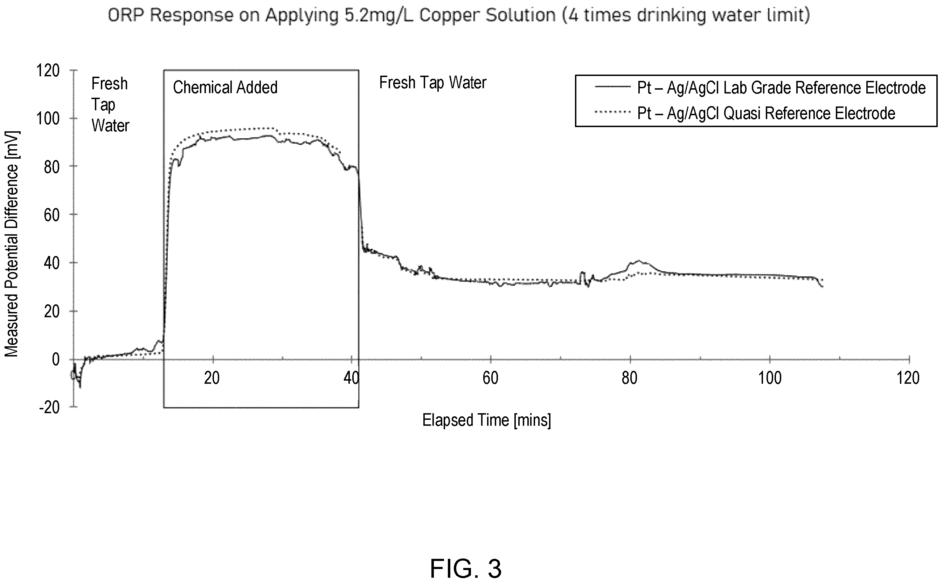

Figure 3 show a measurement illustrating that the Ag/AgCI quasi-reference electrode 12 (the measurement is plotted as a dotted line) is stable since the potential difference measured between the quasi-reference electrode 12 and the working electrode 13 shows the same absolute time dependence as the potential difference between a laboratory-grade reference electrode (not shown) and a working electrode (not shown) (the measurement is plotted as a solid line) to within +/- 10 mV.

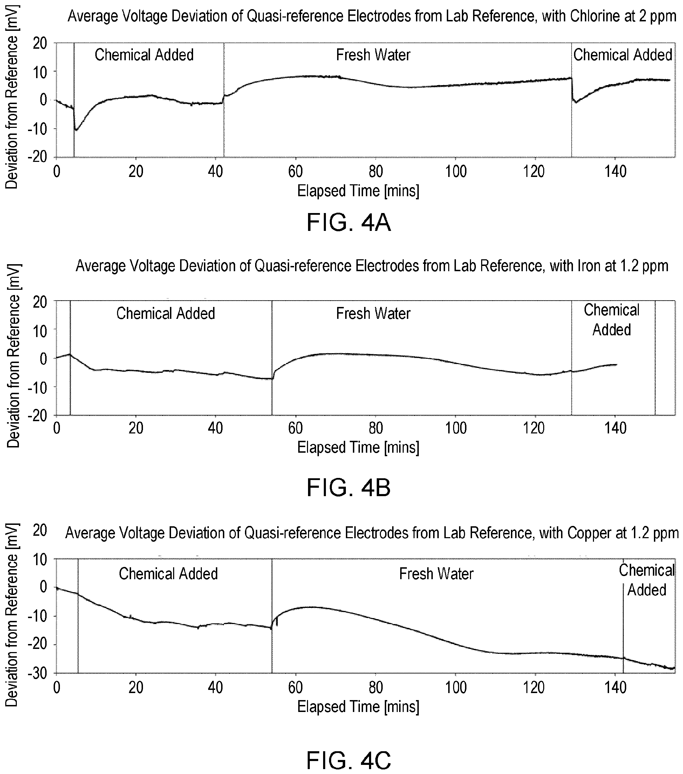

Figures 4A to 4C show the variation of the quasi-reference electrode potential with respect to a laboratory grade reference electrode (Ag/AgCI/KCI(3M)) over the course of three experiments where different chemicals were been introduced into the water flowing through the measurement section 3. The variations are lower than 10 mV over 100 minutes thereby confirming that a silver chloride coated silver wire operates as a quasi-reference electrode.

Figure 4A shows the results in which chlorine at a concentration of 2 ppm is added to the water flowing through the measurement section during the period in the shaded area.

Figure 4B shows the results in which copper at a concentration of 1.3 ppm is added to the water flowing through the measurement section during the period in the shaded area.

Figure 4C shows the results in which iron at a concentration of 1.3 ppm is added to the water flowing through the measurement section during the period in the shaded area.

In all three cases, the drift against a true reference electrode is lower than +/- 10 mV over 100 minutes.

Referring again to Figure 1, the two electrodes 12, 13 may be placed in a separate measurement section 3 and used exclusively to monitor changes in the ORP potential, and are connected to a dedicated electronic circuit 14 which measures the potential difference and produces an output value which can be communicated to a separate control unit 16.

Alternatively, the electrodes 12, 13 may be placed in the measurement section of a flow meter 17 and used exclusively to monitor changes in the ORP potential. The electrodes 12, 13 are connected to a high impedance sampling circuit 14 which measures the potential difference and communicates it to the flow meter main electronics 17. The dedicated electronics 14 can be hosted on a separate PCB or placed on the same PCB used by the water meter electronics 16.

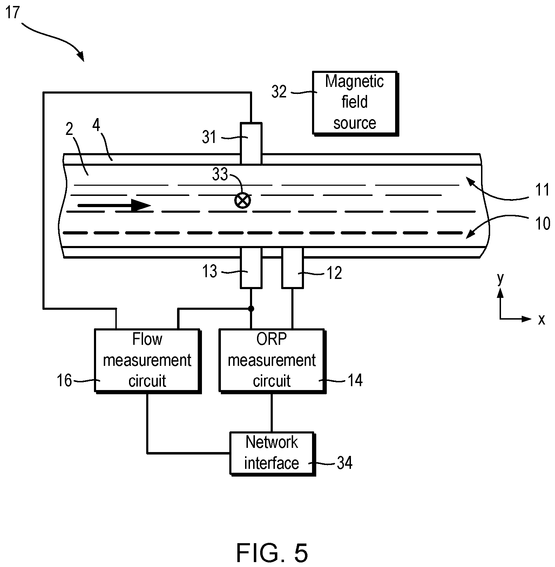

Referring to Figure 5, a water flow meter 17 is shown which is of the electromagnetic flow meter type and which incorporates the ORP monitor 1. The working electrode 13 is also used for the flow measurement section of the flow meter. The water flow meter 17 includes a flow-sensing electrode 31 on the opposite side 11 of the pipe 4 from the working electrode 13. The flow meter 17 also includes a magnetic field source 32, for example, in the form of a coil and a source for driving a current through the coil, for generating a magnetic field 33 across the flow passage 6.

In some embodiments, the flow meter 17 incorporates the ORP monitor 1 which has only two electrodes 12, 13 (for example, disposed on opposite sides of the flow passage 6), and the two electrodes 12, 13 are used for both measuring flow rate and ORP. Accordingly, the ORP measurement circuit 14 and the flow measurement circuit 16 are connected to both electrodes 12, 13. For the flow rate measurement, the direction of the magnetic field 33 can be periodically switched (or "flipped") so as to provide a pulsed voltage signal which switches between a first value and a second value about the offset voltage. The circuitry responsible of measuring the flow rate can be connected to the two electrode using a high pass filter (such as a capacitor in series with the electrode leads), filtering out the DC offset which includes the ORP voltage. ORP can instead be measured by measuring the DC offset voltage with a high input impedance ORP measurement circuit 14 connected via a low pass filter which will filter out the voltage oscillations caused by the magnetic field flipping.

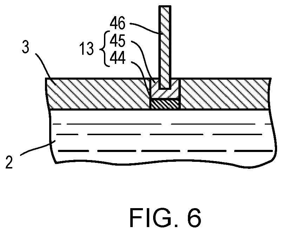

The ORP monitor 1 and the water flow meter 17 are provided with a network interface 34, preferably in the form of a wireless communications network interface. The wireless communications network is preferably a low-power, wide-area (LPWA) network, such as LoRa (RTM), which may communicate directly with a central node 52 (Figure 6). The network interface 34 may be included in control unit 16 of the water flow meter 17.

In another embodiment the quasi-reference electrode consists of a silver (Ag) wire anodised with silver chloride (AgCl) directly immersed in the water flowing through the water meter, in other words, without a porous section.

Referring to Figure 6, the working electrode 13 is shown.

The working electrode 13 forms part of an electrode assembly 41. The electrode assembly 41 comprises a housing, which in this case is the pipe wall 4. The housing 4 has a passage 9 (that is, the aperture) having first and second ends 42, 43.

The working electrode 13 comprises a plug 44 of porous material at least partially disposed within the passage 8 proximate the first end 42 of the passage 9 (that is, the end which is wetted) and an electrically-conductive polymer piece 45 at least partially disposed within the passage 9 and in direct contact with the plug 44. The polymer piece 45 may also serve as a seal preventing fluid from reaching the second end of the passage 9. To provide an alternative seal or to provide an additional seal, the assembly 41 can include an 'O'-ring (not shown) disposed between the plug 44 and electrically-conductive polymer piece 45 which urges against the inner wall of the passage 8.

The plug 44 may be formed of graphite or ceramic. The electrically-conductive polymer piece 45 may be formed from an elastomer, such as silicone or ethylene propylene diene monomer rubber, and particles of electrically-conductive material, such as carbon, carbon black, carbon nanotubes, or silver.

A further metal connector 46 can be inserted into the polymer piece 45 to provide a terminal or connector.

Further details of the electrode can be found in WO 2021/032948 A1 which is incorporated herein by reference.

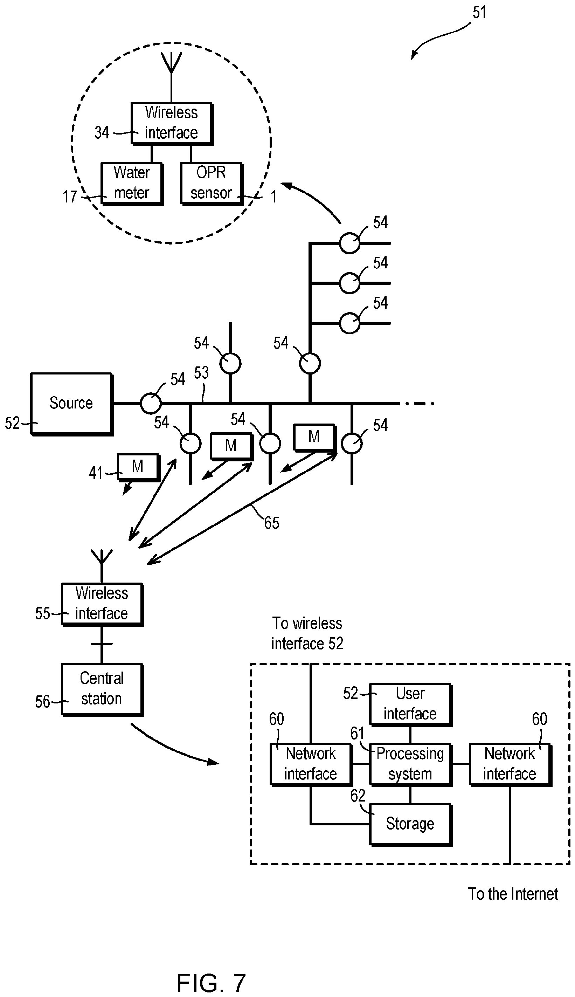

Referring to Figure 7, a drinking water distribution network 51 is shown which includes a source 52 of drinking water, a network of pipes 53, water measurement nodes 54, a wireless access point 55 and a central station 56.

Each water measurement node 54 includes a fiscal water meter 57, a water sensor 1 and a wireless network interface 59.

The central station 56 includes a first network interface 60 for interfacing with the water measurement nodes 54, a processing system 61, storage 62, a user interface 63 and an optional second network interface 64, *e.g*., for interfacing with the Internet.

The sensor 1 can communicate information back to a processing unit or gateway unit (not shown) which reports the information back to a centralised station 56 via wireless links 65 which can then remotely monitor the quality of the water.

The sensor 1 may be mounted inside or next to a smart water meter 57 and is directly connected to the meter electronics, which manages the measurement schedule and is responsible for reporting the measurement value to a centralised system 56.

The lower power consumption, lower cost and the possibility to install the sensor next to an existing or co-installed water meter while sampling the main water stream help to enable a large number of sensors to be deployed at the point of use in a drinking water distribution network 51 thereby providing valuable real-time data on the characteristics of the water being delivered to the end user.

The information allows for a faster response to issues which might cause customer complaints and cause issues with the quality of the delivered water and a better monitoring of network operation which can enable a more flexible operation of the network.

The information collected by the centralised system can be correlated with the geographical position of the water meter node 54 and the known topology of the network so that in case of an event which leads to changes in one of the measured properties the system can estimate the most probable location of the origin of the detected change.

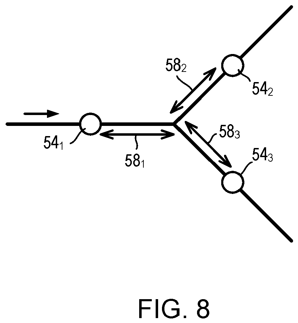

Referring to Figure 8, a simple example of a pipe network is shown in which water flows through a first water measurement node 54₁ into a first pipe section 58₁ which splits into second and third pipe sections 58₂, 58₃ each having a respective node 54₂, 54s. If the first and second water measurement nodes 54₁, 54₂ measure low values of turbidity but the third water measurement node 54s measures a high value of turbidity, then the probable location is in the third pipe section 58s.

The central station 56 can use a model (not shown) to simulate ingress of contaminant. The results can be displayed via the user interface.

The most probable location of the origin of the detected change can be found, for example, by identifying the population of meters which measure a change or drift in the measured property and tracking the section of the network that feeds water to the meter population. Additionally, meters can be clustered based on the magnitude and time evolution of the measured change, to provide an estimate of the pipe section where the issue might be arising to mitigate the effect of false positive readings from single sensors.

The centralised system can also provide an estimate of the severity of the issue affecting the network by correlating the number of meters which detect the change in the measured property with the magnitude of the measured change. This may be used to determine whether urgent action is required.

For example, a slow drift of the measured parameter(s) can indicate a minor issue in the network, so a low priority alarm can be raised, linked to a time estimate for when the parameters will be out of tolerance and maintenance will be required. This can further be connected to the number of meters (hence the portion of the network) being affected by the issue. This way, smaller issues which however affect a large household population could still be considered as a severe issue and dealt with high priority.

Furthermore, the centralised system can also infer the characteristics of the cause of the measured change by correlating the degree of discolouration with the colour of the dissolved material and/or the change in ORP, and/or the change in turbidity, for example distinguishing the ingress of soil through a damage pipe, such as clay or red sand, versus the presence of rust due to pipe corrosion. The information can be fed to the maintenance crews in order to provide them with more accurate instruction on the maintenance operation required, reducing the number of site visits.

As an example, a discolouration value indicating the presence of red/brown dissolved matter can be correlated with the change in ORP. The presence of rust has shown to induce a relatively small change in ORP potential, while the ingress of soil will quickly exhaust the chlorine disinfectant in the water supply causing a sharp decrease in ORP potential. So based on the change in ORP potential it is possible to discriminate between ingress of soil particles in a broken pipe against the presence of iron oxide due to corrosion in the pipe. The information can then be used to direct the maintenance effort based on the advanced knowledge on the type of issue being experienced in the network.

As an additional example, when an increase in turbidity is observed in a section of the network, this can be monitored against the measured ORP value. Suspended particles should not change ORP significantly, however they sustain bacterial growth. Bacterial proliferation will use up the disinfectant in the water supply, so that the issue can be considered a low or high priority based on the observed changed in ORP: low priority if no change in ORP is observed, high priority if a reduction in ORP potential is observed.

It will be appreciated that many modifications may be made to the embodiments hereinbefore described.

## Claims

1. Apparatus, comprising:
· a measurement section (3), comprising:
- a flow tube (4) having a flow passage (6);
· a quasi-reference electrode (12) in fluid communication with the flow passage;
· a working electrode (13) in fluid communication with the flow passage;
· a circuit (14) configured to measure the potential difference between the quasi-reference electrode and the working electrode; and
· an interface (15) for outputting the potential difference;
wherein the quasi-reference electrode comprises:
· a coated metal electrode consisting of a metal electrode having a surface which at least part of which is coated with a layer of an insoluble salt of the metal.

2. The apparatus of claim 1, wherein the metal is silver and, optionally, the insoluble salt is silver chloride.

3. The apparatus of claim 1 or 2, wherein the quasi-reference electrode further comprises:
· a porous element,
wherein the porous element is interposed between the coated metal electrode and the flow tube so as to provide a fluid path between the flow tube and the coated metal electrode.

4. The apparatus of claim 3, wherein the porous element is a ceramic or graphite.

5. The apparatus of claim 1 or any one of claims 2 to 4, wherein the working electrode comprises:
· an inert, electrically-conductive material electrode at least partially disposed within a passage at an end which is proximate to the flow passage.

6. The apparatus of claim 5, wherein the inert, electrically-conductive material comprises a noble metal, such as platinum or gold, or carbon, such as graphite.

7. The apparatus of claim 5 or 6, wherein the inert, electrically-conductive material electrode is porous.

8. The apparatus of claim 7, further comprising:
· an electrically-conductive connector in direct contact with the electrically-conductive material electrode, wherein the inert, electrically-conductive material electrode is interposed between the electrically-conductive connector and the flow passage.

9. The apparatus of claim 8, wherein the electrically-conductive connector comprises, or is formed of, an electrically-conductive polymer, optionally wherein the electrically-conductive connector is configured to provide a fluid-tight seal in the passage.

10. The apparatus of claim 1 or 2, wherein the flow tube includes an aperture and wherein the quasi-reference electrode and the working electrode are disposed in the aperture.

11. The apparatus of claim 1 or any one of claims 2 to 10, further comprising:
· a further electrode in fluid communication with the flow passage, wherein the working electrode and further electrode are spaced apart for electromagnetic flow sensing.

12. The apparatus of claim 11,
wherein the working electrode comprising an inert, electrically-conductive material electrode and the further electrode comprising an inert, electrically-conductive material electrode, and the working electrode and the and further electrodes are disposed on opposite sides of the flow tube, or
wherein the quasi-reference electrode is a first quasi-reference electrode and the further electrode is a second quasi-reference electrode comprising a coated metal electrode consisting of a metal electrode having a surface which at least part of which is coated with a layer of an insoluble salt of the metal, wherein the first and second quasi-reference electrodes are disposed on opposite sides of the flow tube.

13. The apparatus of claim 1 or any one of claims 2 to 12, wherein the working electrode and the quasi-reference electrode are disposed on opposite sides of the flow tube.

14. The apparatus of claim 1 or any one of claims 2 to 13, wherein the fluid is a liquid, and optionally, the liquid is water.

15. A water meter comprising:
· the apparatus of claim 1 or any one of claims 2 to 14; and
· apparatus for measuring fluid flow through the flow tube,
optionally wherein the apparatus for measuring fluid flow through the flow tube is an electromagnetic flow type water meter.
